# EUROPEAN PATENT APPLICATION

(11) **EP 1 897 512 A2**
(43) Date of publication of application: **12.03.2008**
(21) Application number: 07253592.5
(22) Date of filing: 10.09.2007
(51) Int. Cl.: A61B 19/00, F03G 5/02

(54) **Locating an orthopaedic medical device**

(30) Priority: 11.09.2006 US 530572
(71) Applicant: DePuy Products, Inc., Warsaw, IN 46581 (US)
(72) Inventor: Caylor, Edward J. III, Fort Wayne IN 46814 (US)
(74) Representative: Belcher, Simon James

(57) **Abstract**

A system for determining a location of an orthopaedic medical device includes a controller and an array of antennae. The array of antennae includes a number of coplanar antennae and one or more additional antennae positioned non-coplanar relative to the coplanar antennae. The coplanar and non-coplanar antennae may be directional antennae. The orthopaedic medical device includes a wireless transmitter circuit configured to transmit a serial number of the device on a predetermined carrier frequency. The orthopaedic medical device may be coupled to a bone of the patient, an orthopaedic implant, or an orthopaedic surgical tool. The controller is electrically coupled to the array of antennae and configured to determine a location of the orthopaedic medical device based on output signals received from the antennae in response to the modulated wireless signal.

## Description

This invention relates to computer assisted surgery systems for use in the performance of orthopaedic procedures.

There is an increasing adoption of minimally invasive orthopaedic procedures. Because such surgical procedures generally restrict the surgeon's ability to see the operative area, surgeons are increasingly relying on computer systems, such as computer assisted orthopaedic surgery (CAOS) systems, to assist in the surgical operation.

Computer assisted orthopaedic surgery (CAOS) systems assist surgeons in the performance of orthopaedic surgical procedures by, for example, displaying images show in surgical steps of the surgical procedure being performed and rendered images of the relevant bones of the patient. Additionally, computer assisted orthopaedic surgery (CAOS) systems provide surgical navigation for the surgeon by tracking and displaying the position of the patient's bones, implants, and/or surgical tools. To do so, in typical computer assisted orthopaedic surgery (CAOS) systems, one or more fiducial markers are attached to the patent's bones, the orthopaedic implant, and/or the surgical tools. Based on the positioning of the fiducial markers, the positioning of the relevant bones, orthopaedic implant, and/or surgical tools is determined and displayed to the surgeon.

In one aspect, the invention provides a computer assisted orthopaedic surgery system which includes a first orthopaedic medical device and/or a second orthopaedic medical device. The first orthopaedic medical device may include a first wireless transmitter. The first wireless transmitter may be configured to transmit a first wireless signal using a predetermined carrier frequency. Similarly, the second orthopaedic medical device may include a second wireless transmitter that is configured to transmit a second wireless signal using the predetermined carrier frequency. In some embodiments, the first orthopaedic medical device and/or the second orthopaedic medical device may be coupled to an orthopaedic implant, an orthopaedic surgical tool, and/or a bone of a patient. Additionally, the first orthopaedic medical device may be configured to transmit the first wireless signal at a first pulse repetition frequency different from a second pulse repetition frequency used by the second wireless signal to transmit the second wireless signal. The first wireless signal may include a serial number of the first orthopaedic medical device. Similarly, the second wireless signal may includes a serial number of the second orthopaedic medical device.

The computer assisted orthopaedic surgery system may also include a plurality of antennae. The plurality of antennae may include a plurality of first antennae and a second antenna. The plurality of first antennae may each be positioned substantially coplanar with each other and the second antenna may be positioned non-coplanar with respect to the plurality of first antennae. In some embodiments, the plurality of first antennae and the second antenna may be spiral directional antennae. Additionally, the plurality of first antennae may be positioned such that a boresight of each first antenna is directed toward a common volume of space. Similarly, the second antenna may be positioned such that a boresight of the second antenna is directed toward the common volume of space.

The computer assisted orthopaedic surgery system may also include a controller electrically coupled to the plurality of antennae. The controller may be configured to receive first output signals from the plurality of antennae in response to the first wireless signal and second output signals from the plurality of antennae in response to the second wireless signal. The controller may also be configured to demodulate the first and second output signals. In addition, the controller may be configured to determine a location of the first orthopaedic medical device based on the demodulated first output signals and a location of the second orthopaedic medical device based on the demodulated second output signals. To do so, the controller may be configured to, for example, compare the demodulated first output signals to determine the location of the first orthopaedic medical device and compare the demodulated second output signals to determine the location of the second orthopaedic medical device. For example, the controller may the location of the first and second orthopaedic medical devices by using a radio frequency direction finding algorithm. Further, in some embodiments, the computer assisted orthopaedic surgery system may includes a display device electrically coupled to the controller and the controller may be configured to display indicia of the location of the first and second orthopaedic medical devices on the display screen.

The system of the invention can be used in a method for determining a location of an orthopaedic medical device having a wireless transmitter associated therewith may include receiving a modulated wireless signal from the wireless transmitter with a plurality of coplanar antennae and with an antenna positioned non-coplanar with respect to the coplanar antennae. In some embodiments, the first and second antennae may be spiral directional antennae. The method may also include receiving output signals from each of the antennae in response to the modulated wireless signal and demodulating the output signals. Additionally, the method may include determining data indicative of the location of the orthopaedic medical device based on the demodulated output signals. The data indicative of the location of the orthopaedic medical device may be determined by comparing the demodulated output signals. For example, the amplitude of the demodulated output signals, the phase of the demodulated output signals, the Doppler frequency shift of the demodulated output signals, and/or the differential time of arrival of the demodulated output signals may be compared. The method may further include displaying indicia of the location of the orthopaedic medical device on a display device based on the determining step.

Accordingly, in another aspect, the invention provides a method for determining a location of an orthopaedic medical device having a wireless transmitter associated therewith, the method comprising:
receiving a modulated wireless signal from the wireless transmitter with a plurality of coplanar antennae;
receiving the modulated wireless signal with an antenna positioned non-coplanar with respect to the coplanar antennae;
receiving output signals from each of the antennae;
demodulating the output signals; and
determining data indicative of the location of the orthopaedic medical device based on the demodulated output signals.

In a further aspect, the invention provides a computer assisted orthopaedic surgery system which includes an orthopaedic medical device having a wireless transmitter that is configured to transmit a modulated wireless signal. The modulated wireless signal may include, for example, a serial number of the orthopaedic medical device. The computer assisted orthopaedic surgery system may also include a plurality of first antennae, each of which is positioned substantially coplanar with each other, and a second antenna positioned non-coplanar with respect to the plurality of first antennae. Additionally, the computer assisted orthopaedic surgery system may include a controller electrically coupled to the plurality of first antennae and the second antenna. The controller may be configured to receive output signals from the plurality of first antennae and the second antenna in response to the modulated wireless signal, demodulate the output signals, and determine the location of the orthopaedic medical device based on the demodulated output signals. The controller may determine the location of the orthopaedic medical device by, for example, using a radio frequency direction finding algorithm.

In yet another aspect, the invention provides an implantable orthopaedic medical device for use in determining a location of a bone of a patient which includes a housing and an antenna coil positioned in the housing. The implantable orthopaedic medical device may also include a memory device positioned in the housing. The memory device may have stored therein a serial number associated with the implantable orthopaedic medical device. The implantable orthopaedic medical device may also include a transmitter circuit positioned in the housing and electrically coupled to the antenna coil and the memory device. The transmitter circuit may be configured to transmit the serial number on a predetermined carrier frequency at a predetermined pulse repetition frequency using the antenna coil. The implantable orthopaedic medical device may also include a switching circuit coupled to the antenna coil and the transmitter circuit. The switching circuit may be operable to selectively electrically connect the antenna coil to a power terminal of the transmitter circuit or an output terminal of the transmitter circuit. Additionally, the implantable orthopaedic medical device may include a power coil electrically coupled to the transmitter circuit. The power coil may be configured to be inductively coupled to a power source external to the patient to supply power to the transmitter circuit.

Embodiments of the invention will now be described by way of example with reference to the accompanying drawings, in which:
FIG. 1 is a perspective view of a computer assisted orthopaedic surgery (CAOS) system;
FIG. 2 is a simplified diagram of the CAOS system of FIG. 1;
FIG. 3 is a perspective view of a bone locator tool;
FIG. 4 is a perspective view of a registration tool for use in the system of FIG. 1;
FIG. 5 is a perspective view of an orthopaedic surgical tool for use with the system of FIG. 1;
FIG. 6 is a simplified diagram of another computer assisted orthopaedic surgery (CAOS) system;
FIG. 7 is a simplified diagram of one embodiment an orthopaedic medical device of the CAOS system of FIG. 6;
FIG. 8 is a simplified diagram of another embodiment of an orthopaedic medical device of the CAOS system of FIG. 6;
FIG. 9 is a perspective view of one embodiment of a housing of the orthopaedic medical device of FIG. 7 and/or 8;
FIG. 10 is a perspective view of an antenna array of the CAOS system of FIG. 6 incorporated into an orthopaedic surgery operating room;
FIG. 11 is a plan view of a first portion of the antenna array of FIG. 10;
FIG. 12 is a cross-sectional view of a second portion of the antenna array of FIG. 10;
FIG. 13 is a cross-sectional view of another embodiment of the second portion of the antenna array of FIG. 10;
FIG. 14 is a simplified flowchart of an algorithm executed by the computer assisted orthopaedic surgery (CAOS) system of FIG. 6;
FIG. 15 is a simplified diagram of a system for monitoring kinematic motion of a patient;

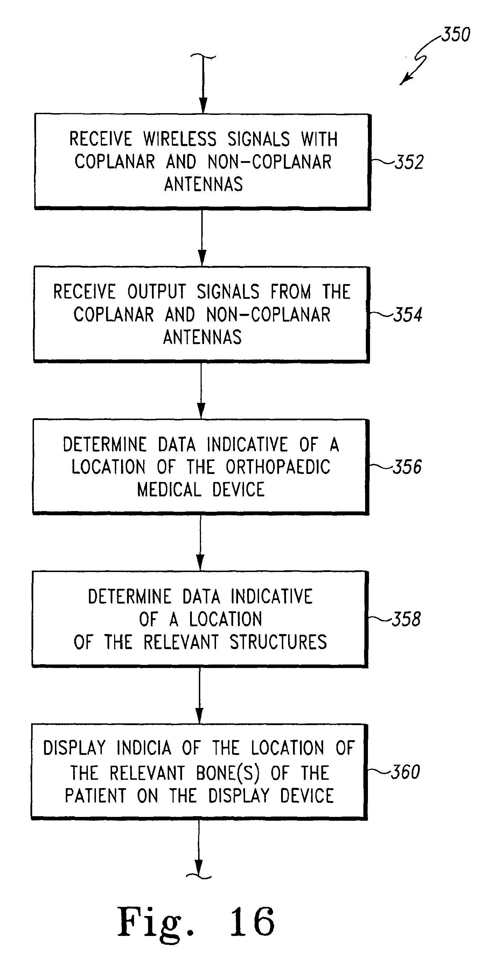

FIG. 16 is a simplified flowchart of an algorithm executed by the system of FIG. 15;
FIG. 17 is a perspective view of one embodiment of an patient exercise apparatus of the system of FIG. 15;
FIG. 18 is a perspective view of another embodiment of a patient exercise apparatus of the system of FIG. 15;
FIG. 19 is a simplified diagram of another embodiment an orthopaedic medical device of the CAOS system of FIG. 6;
FIG. 20 is a simplified diagram of another embodiment of an orthopaedic medical device of the CAOS system of FIG. 6; and
FIG. 21 is a simplified flowchart of an algorithm that is executed by the computer assisted orthopaedic surgery (CAOS) system of FIG. 6 and/or the system of FIG. 15.

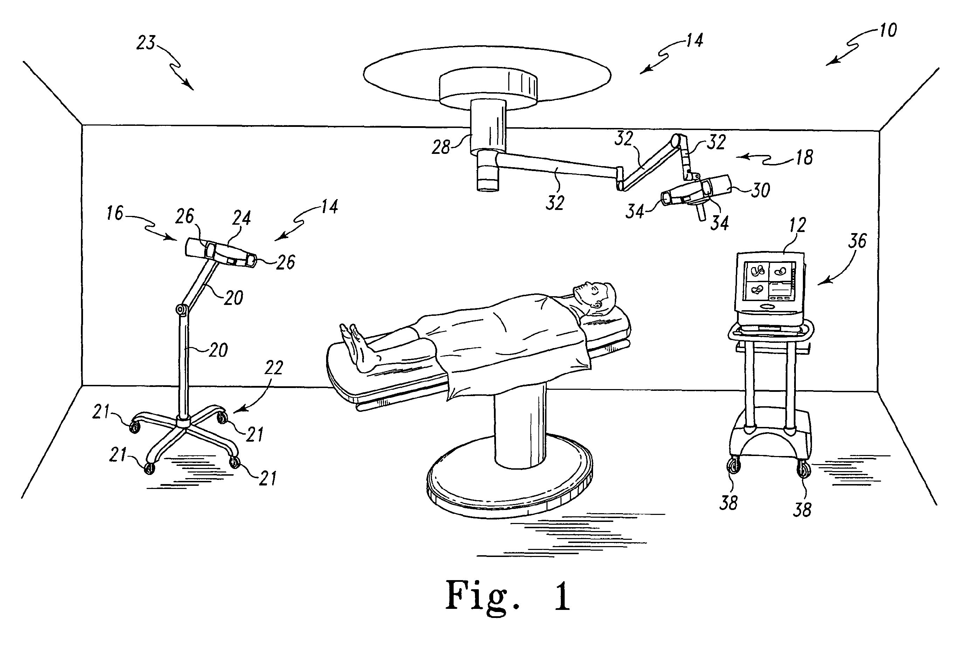

Referring to the drawings, FIG. 1 shows a computer assisted orthopaedic surgery (CAOS) system 10 which includes a computer 12 and a camera unit 14. The CAOS system 10 may be embodied as any type of computer assisted orthopaedic surgery system. The CAOS system 10 can be embodied as one or more computer assisted orthopaedic surgery systems commercially available from DePuy Orthopaedics, Inc. of Warsaw, Indiana and/or one or more computer assisted orthopaedic surgery systems commercially available from BrainLAB of Heimstetten, Germany. The camera unit 14 may be embodied as a mobile camera unit 16 or a fixed camera unit 18. In some embodiments, the system 10 may include both types of camera units 16, 18. The mobile camera unit 16 includes a stand 20 coupled with a base 22. The base 22 may include a number of wheels 21 to allow the mobile camera unit 16 to be repositioned within a hospital room 23. The mobile camera unit 16 includes a camera head 24. The camera head 24 includes two cameras 26. The camera head 24 may be positioned relative to the stand 20 such that the field of view of the cameras 26 may be adjusted. The fixed camera unit 18 is similar to the mobile camera unit 16 and includes a base 28, a camera head 30, and an arm 32 coupling the camera head 30 with the base 28. In some embodiments, other peripherals, such as display screens, lights, and the like, may also be coupled with the base 28. The camera head 30 includes two cameras 34. The fixed camera unit 18 may be coupled to a ceiling, as shown for example in FIG. 1, or a wall of the hospital room. Similar to the camera head 24 of the camera unit 16, the camera head 30 may be positioned relative to the arm 32 such that the field of view of the cameras 34 may be adjusted. The camera units 14, 16, 18 are communicatively coupled with the computer 12. The computer 12 may be mounted on or otherwise coupled with a cart 36 having a number of wheels 38 to allow the computer 12 to be positioned near the surgeon during the performance of the orthopaedic surgical procedure.

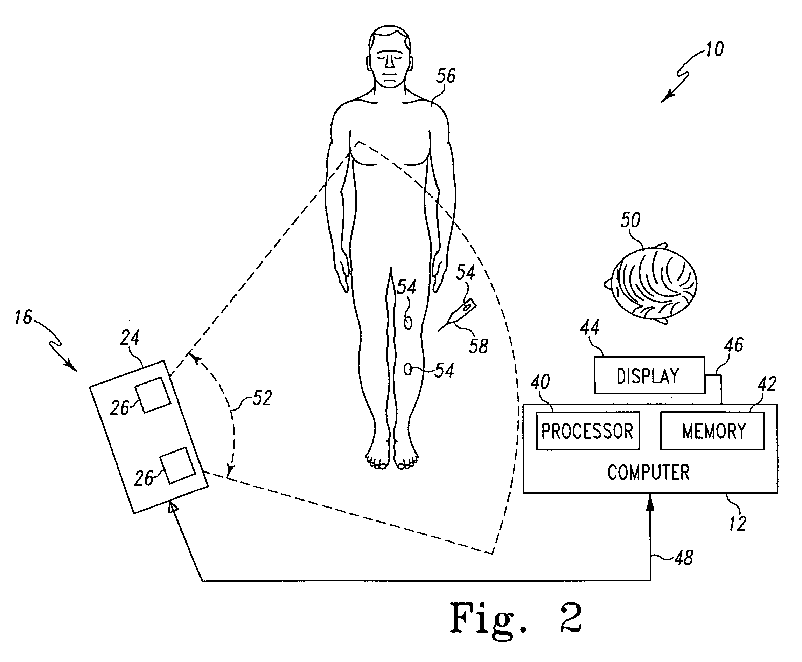

FIG. 2 shows the computer 12 which includes a processor 40 and a memory device 42. The processor 40 may be embodied as any type of processor including, for example, discrete processing circuitry (for example a collection of logic devices), general purpose integrated circuit(s), and/or application specific integrated circuit(s) (ASICs). The memory device 42 may be embodied as any type of memory device and may include one or more memory types, such as, random access memory (RAM) and/or read-only memory (ROM). In addition, the computer 12 may include other devices and circuitry typically found in a computer for performing the functions described herein such as, for example, a hard drive, input/output circuitry, and the like.

The computer 12 is communicatively coupled with a display device 44 via a communication link 46. Although shown in FIG. 2 as separate from the computer 12, the display device 44 may form a portion of the computer 12 in some embodiments. Additionally, in some embodiments, the display device 44 or an additional display device may be positioned away from the computer 12. For example, the display device 44 may be coupled with the ceiling or wall of the operating room in which the orthopaedic surgical procedure is to be performed. Additionally or alternatively, the display device 44 may be embodied as a virtual display such as a holographic display, a body mounted display such as a heads-up display, or the like. The computer 12 may also be coupled with a number of input devices such as a keyboard and/or a mouse for providing data input to the computer 12. However, in the embodiment which is shown in the FIG. 2, the display device 44 is a touch-screen display device capable of receiving inputs from an orthopaedic surgeon 50. That is, the surgeon 50 can provide input data to the computer 12, such as making a selection from a number of on-screen choices, by simply touching the screen of the display device 44.

The computer 12 is also connected to the camera unit 16 (and/or 18) via a communication link 48. Preferably, the communication link 48 is a wired communication link but, in some embodiments, may be embodied as a wireless communication link. In embodiments in which the communication link 48 is a wireless signal path, the camera unit 16 and the computer 12 include wireless transceivers such that the computer 12 and camera unit 16 can transmit and receive data (for example image data). Although only the mobile camera unit 16 is shown in FIG. 2, it should be appreciated that the fixed camera unit 18 may alternatively be used or may be used in addition to the mobile camera unit 16.

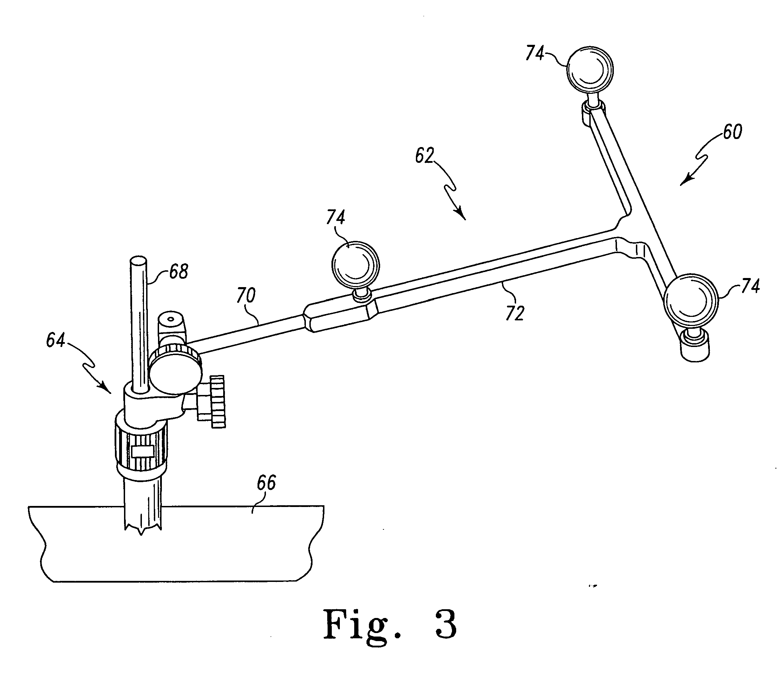

The CAOS system 10 may also include a number of sensors or sensor arrays 54 which may be coupled the relevant bones of a patient 56 and/or with orthopaedic surgical tools 58. For example, as shown in FIG. 3, a tibial array 60 includes a sensor array 62 and bone clamp 64. The bone clamp 64 is configured to be coupled with a tibia bone 66 of the patient 56 using a Schantz pin 68, but other types of bone clamps may be used. The sensor array 62 is coupled with the bone clamp 64 via an extension arm 70. The sensor array 62 includes a frame 72 and three reflective elements or sensors 74. The reflective elements 74 are embodied as spheres in the described embodiment, but may have other geometric shapes in other embodiments. Additionally, in other embodiments sensor arrays having more than three reflective elements may be used. The reflective elements 74 are positioned in a predefined configuration that allows the computer 12 to determine the identity of the tibial array 60 based on the configuration. That is, when the tibial array 60 is positioned in a field of view 52 of the camera head 24, as shown in FIG. 2, the computer 12 is configured to determine the identity of the tibial array 60 based on the images received from the camera head 24. Additionally, based on the relative position of the reflective elements 74, the computer 12 is configured to determine the location and orientation of the tibial array 60 and, accordingly, the tibia 66 to which the array 60 is coupled.

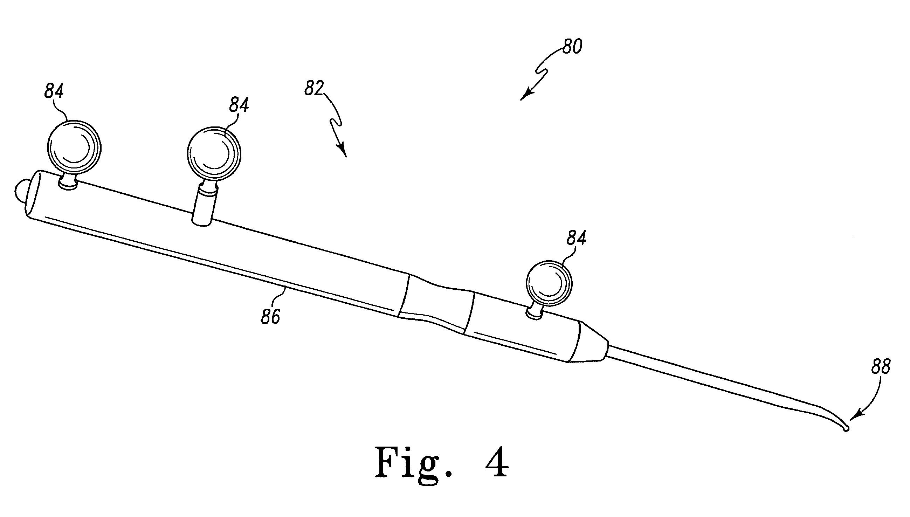

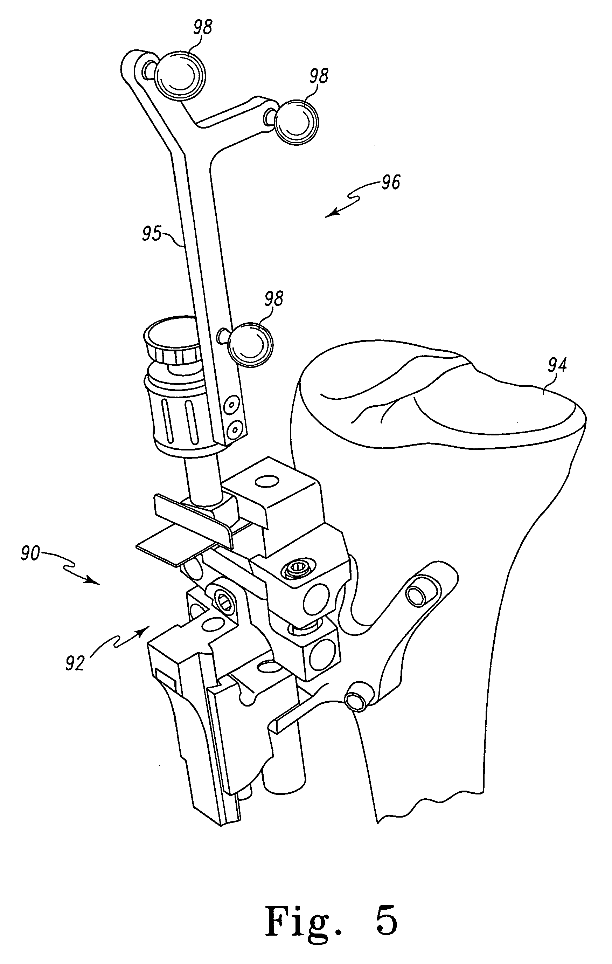

Sensor arrays may also be coupled to other surgical tools. For example, a registration tool 80, as shown in FIG. 4, is used to register points of a bone of the patient. The registration tool 80 includes a sensor array 82 having three reflective elements 84 coupled with a handle 86 of the tool 80. The registration tool 80 also includes pointer end 88 that is used to register points of a bone. The reflective elements 84 are also positioned in a configuration that allows the computer 12 to determine the identity of the registration tool 80 and its relative location (that is, the location of the pointer end 88). Additionally, sensor arrays may be used on other surgical tools such as a tibial resection jig 90, as shown in FIG. 5. The jig 90 includes a resection guide portion 92 that is coupled with a tibia bone 94 at a location of the bone 94 that is to be resected. The jig 90 includes a sensor array 96 that is coupled with the portion 92 via a frame 95. The sensor array 96 includes three reflective elements 98 that are positioned in a configuration that allows the computer 12 to determine the identity of the jig 90 and its relative location (for example with respect to the tibia bone 94).

The CAOS system 10 may be used by the orthopaedic surgeon 50 to assist in any type of orthopaedic surgical procedure including, for example, a total knee replacement procedure. To do so, the computer 12 and/or the display device 44 are positioned within the view of the surgeon 50. As discussed above, the computer 12 may be coupled with a movable cart 36 to facilitate such positioning. The camera unit 16 (and/or camera unit 18) is positioned such that the field of view 52 of the camera head 24 covers the portion of a patient 56 upon which the orthopaedic surgical procedure is to be performed, as shown in FIG. 2.

During the performance of the orthopaedic surgical procedure, the computer 12 of the CAOS system 10 is programmed or otherwise configured to display images of the individual surgical procedure steps which form the orthopaedic surgical procedure being performed. The images may be graphically rendered images or graphically enhanced photographic images. For example, the images may include three dimensional rendered images of the relevant anatomical portions of a patient. The surgeon 50 may interact with the computer 12 to display the images of the various surgical steps in sequential order. In addition, the surgeon may interact with the computer 12 to view previously displayed images of surgical steps, selectively view images, instruct the computer 12 to render the anatomical result of a proposed surgical step or procedure, or perform other surgical related functions: For example, the surgeon may view rendered images of the resulting bone structure of different bone resection procedures. In this way, the CAOS system 10 provides a surgical "walk-through" for the surgeon 50 to follow while performing the orthopaedic surgical procedure.

In some embodiments, the surgeon 50 may also interact with the computer 12 to control various devices of the system 10. For example, the surgeon 50 may interact with the system 10 to control user preferences or settings of the display device 44. Further, the computer 12 may prompt the surgeon 50 for responses. For example, the computer 12 may prompt the surgeon to inquire if the surgeon has completed the current surgical step, if the surgeon would like to view other images, and the like.

The camera unit 16 and the computer 12 also cooperate to provide the surgeon with navigational data during the orthopaedic surgical procedure. That is, the computer 12 determines and displays the location of the relevant bones and the surgical tools 58 based on the data (for example images) received from the camera head 24 via the communication link 48. To do so, the computer 12 compares the image data received from each of the cameras 26 and determines the location and orientation of the bones and tools 58 based on the relative location and orientation of the sensor arrays 54, 62, 82, 96. The navigational data displayed to the surgeon 50 is continually updated. In this way, the CAOS system 10 provides visual feedback of the locations of relevant bones and surgical tools for the surgeon 50 to monitor while performing the orthopaedic surgical procedure.

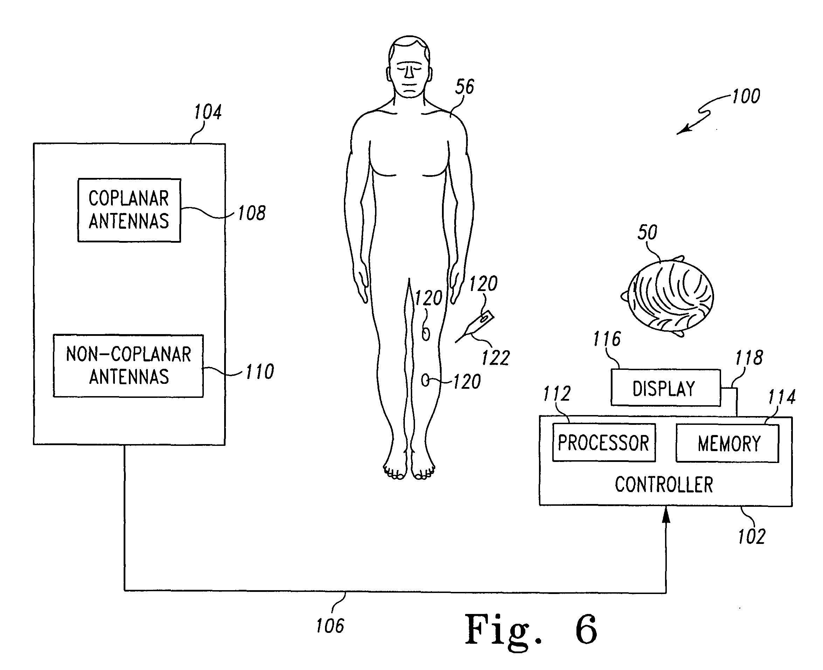

Referring now to FIG. 6, in another embodiment, a computer assisted orthopaedic surgery (CAOS) system 100 includes a controller 102 and an antenna array 104. The controller 102 is electrically coupled to the antenna array 104 via a number of communication links 106. The communication links 106 may be embodied as any type of communication links capable of facilitating electrical communication between the controller 102 and the antenna array 104. For example, the communication links may be embodied as any number of wires, cables, or the like.

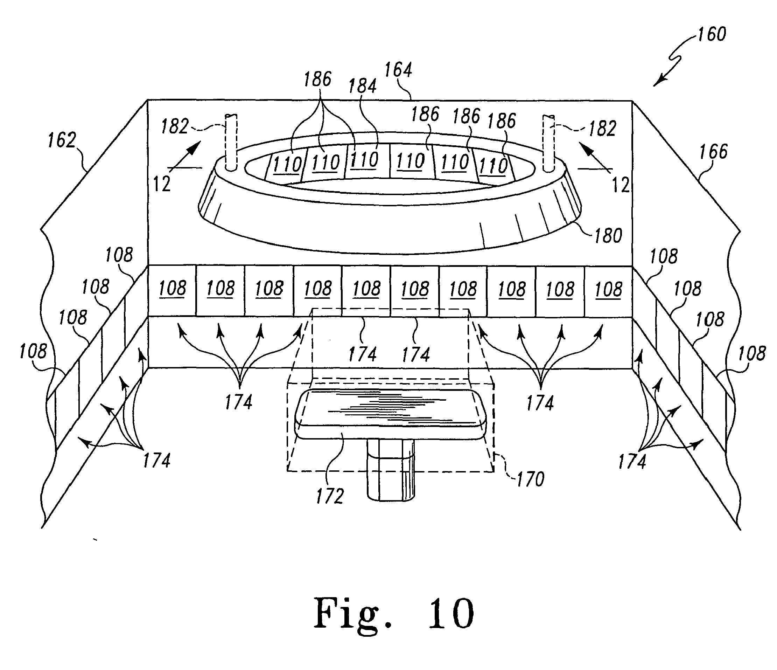

The antenna array 104 includes a number of coplanar antennae 108 and a number of non-coplanar antennae 110 (with respect to the coplanar antennae 108 as discussed in more detail below with reference to FIG. 10). In one embodiment, the antennae 108, 110 are directional antennae having a radiation/receiving pattern that is not omni-directional. For example, the antennae 108, 110 may be uni-directional antennae. In one particular embodiment, the antennae 108, 110 are spiral directional antennae. The directivity of each directional antenna 108, 110 is defined by the beamwidth the antenna 108, 110, which is defined about the boresight of the antenna 108, 110. The boresight of the antenna 108, 110 typically corresponds to a physical axis of the antenna and is defined as the axis of the antenna 108, 110 along which the gain of the antenna 108, 110 is greatest. As such, the antennae 108, 110 are sensitive to signals generated by sources positioned in the beamwidth of the antennae. Conversely, signals incoming toward the antennae 108, 110 from sources outside of the beamwidth of the antennae 108, 110 are substantially attenuated.

The controller 102 includes a processor 112 and a memory device 114. The processor 112 may be embodied as any type of processor including, for example, discrete processing circuitry (for example a collection of logic devices), general purpose integrated circuit(s), and/or application specific integrated circuit(s) (ASICs). The memory device 114 may be embodied as any type of memory device and may include one or more memory types, such as, random access memory (RAM) and/or read-only memory (ROM). In addition, the controller 102 may include other devices and circuitry typically found in a computer for performing the functions described herein such as, for example, a hard drive, input/output circuitry, and the like.

The controller 102 is connected to a display device 116 via a communication link 118. Although shown in FIG. 6 as separate from the computer 102, the display device 116 may form a portion of the controller 102 in some embodiments. Additionally, in some embodiments, the display device 116 or an additional display device may be positioned away from the controller 102. For example, the display device 116 may be coupled to the ceiling or wall of the operating room in which the orthopaedic surgical procedure is to be performed. Additionally or alternatively, the display device 116 may be embodied as a virtual display such as a holographic display, a body mounted display such as a heads-up display, or the like. The controller 102 may also be coupled with a number of input devices such as a keyboard and/or a mouse for providing data input to the controller 102. However, in the embodiment shown in the drawings, the display device 116 is a touch-screen display device capable of receiving inputs from the orthopaedic surgeon 50 similar to the display device 44 described above with reference to FIG. 2. That is, the surgeon 50 can provide input data to the controller 102, such as making a selection from a number of on-screen choices, by simply touching the screen of the display device 116.

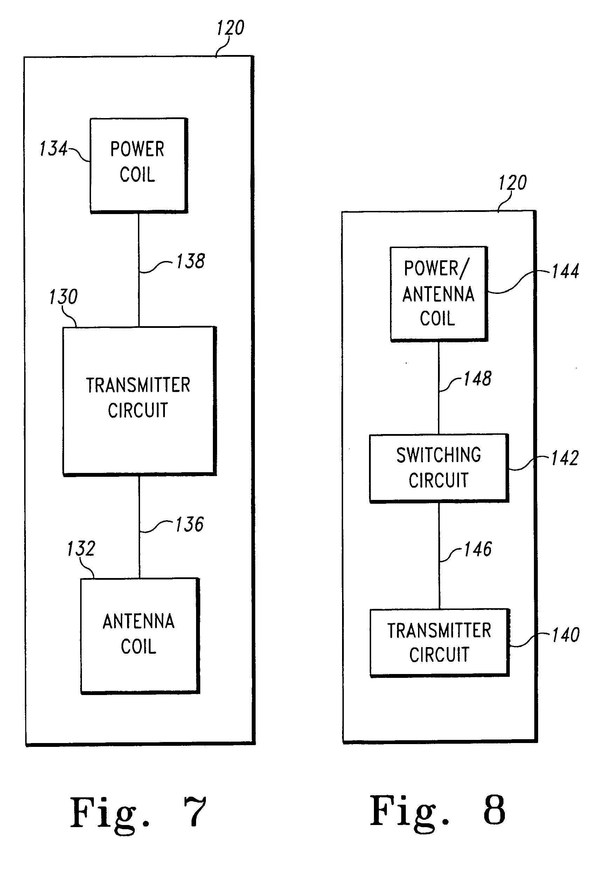

The computer assisted orthopaedic surgery (CAOS) system 100 may also include a number of orthopaedic medical devices 120. The orthopaedic medical devices 120 may be coupled to relevant bones of the patient 56, to orthopaedic surgical tools 122, and/or to orthopaedic implants. As discussed in more detail below with reference to FIGS. 7 and 8, the orthopaedic medical devices 120 transmit a wireless signal that is received by the antenna array 104. In one particular embodiment, the wireless signal is a non-modulated wireless signal of a predetermined frequency. In embodiments in which more than one orthopaedic medical device 120 is used, each orthopaedic medical device 120 may transmit a wireless signal (for example a non-modulated wireless signal) at a different frequency with respect to each other. Alternatively, each orthopaedic medical device 120 may transmit a wireless signal at different pulse repetition frequencies (PRF). That is, each orthopaedic medical device 120 may be configured to transmit a wireless signal having pulses of the same carrier frequency but at different repetition rates.

FIG. 7 shows an orthopaedic medical device 120 which includes a transmitter circuit 130, an antenna coil 132, and a power coil 134. The transmitter circuit 130 is connected to the antenna coil 132 via a number of communication links 136 and to the power coil 134 via a number of communication links 138. The communication links 136, 138 may be embodied as any type of communication link capable of facilitating communication between the transmitter circuit 130 and the antenna coil 132 and power coil 134, respectively. For example, the communication links 136, 138 may be embodied as wires, cables, printed circuit board (PCB) traces, fibre optic cables, or the like. The transmitter circuit 130 may be embodied as or include any type of transmitter circuitry capable of generating a wireless signal at a predetermined frequency. For example, the transmitter circuit 130 may be embodied as a simple inductor-capacitor (LC) circuit or a crystal oscillator circuit and associated circuitry.

As described above, the transmitter circuit 130 may be configured to transmit a wireless signal at a predetermined frequency or a predetermined pulse repetition frequency. In some embodiments, the wireless signal generated by the transmitter circuit 130 is a non-modulated wireless signal. That is, the wireless signal does not include other signals (for example data signals) embedded or modulated in the predetermined carrier frequency. The predetermined frequency of the wireless signal may be any frequency receivable by the antenna array 104. In one embodiment, the transmitter circuit 130 is configured to transmit wireless signals in the very-high frequency (VHF) band or ultra-high frequency (UHF) band. Because the orthopaedic medical device 120 of FIG. 7 does not require sensors or additional circuitry to modulate data from such sensors on the predetermined frequency, the overall size of the orthopaedic medical device 120 may be reduced compared to typical orthopaedic medical devices used for determining the location of patient's bones, orthopaedic implants, or orthopaedic surgical tools. Such reduction in the size of the orthopaedic medical device 120 may improve the orthopaedic surgical procedure by allowing, for example, smaller access incisions in the patient 50.

The transmitter circuit 130 receives power via the power coil 134. The power coil 134 is configured to be inductively coupled to a power source (not shown) external to the patient. The power coil 134 may include any number of individual coils. For example, the power coil 134 may include a single coil that is inductively coupled to the external power source by positioning the external power source near the skin of the patient such that the power coil 134 lies within an alternating current (AC) magnetic field generated by the external power source. In other embodiments, the power coil 134 includes more than a single coil to thereby improve the inductive coupling of the power coil 134 and the external power source. That is, because the amount of inductive coupling of the power coil 134 and the external power source is dependent upon the alignment of the power coil 134 and the magnetic field generated by the external power source, a power coil having multiple coils at different orientations decreases the likelihood of poor inductive coupling with the external power source. For example, in one embodiment, the power coil 134 is embodied as three separate coils positioned orthogonally with respect to each other. The external power source may be embodied as any type of power source capable of inductively coupling with the power coil 134 and generating a current therein. In one embodiment, the external power source includes two patches which can be provided on the skin of the patient in the vicinity of the orthopaedic medical device 120. The patches each include a Helmholtz-like coil and are powered such that the Helmholtz coils produce an isotropic magnetic field, which is received by the power coil 134.

Referring now to FIG. 8, in another embodiment, the orthopaedic medical device 120 includes a transmitter circuit 140, a switching circuit 142, and a power/antenna coil 144. The transmitter circuit 140 is communicatively coupled to the switching circuit 142 via a number of communication links 146. The switching circuit 142 is coupled to the power/antenna coil 144 via a number of communication links 148. Similar to the communication links 136, 138 described above with reference to FIG. 7, the communication links 146, 148 may be embodied as any type of communication link capable of facilitating communication between the transmitter circuit 140, the switching circuit 142, and the power coil 134. For example, the communication links 146, 148 may be embodied as wires, cables, printed circuit board (PCB) traces, fibre optic cables, or the like. The transmitter circuit 140 is substantially similar to the transmitter 130 described above with reference to FIG. 7 and, as such, may be embodied as or include any type of transmitter circuit capable of transmitting a wireless signal via the power/antenna coil 132. For example, the transmitter circuit 140 may be embodied as a simple inductor-capacitor (LC) circuit or a crystal oscillator circuit and associated circuitry.

Similar to the transmitter circuit 130, the transmitter circuit 140 may be configured to transmit a wireless signal at a predetermined frequency or a predetermined pulse repetition frequency. In some embodiments, the wireless signal generated by the transmitter circuit 140 is a non-modulated wireless signal. Additionally, in one embodiment, the transmitter circuit 130 is configured to transmit wireless signals in the very-high frequency (VHF) band or ultra-high frequency (UHF) band. Again, because the orthopaedic medical device 120 of FIG. 8 does not require sensors or additional circuitry to modulate data from such sensors on the predetermined frequency, the overall size of the orthopaedic medical device 120 may be reduced compared to typical orthopaedic medical devices used for determining the location of patient's bones, orthopaedic implants, or orthopaedic surgical tools.

In the embodiment shown in FIG. 8, the transmitter circuit 140 receives power and transmits a wireless signal using the same coil, the power/antenna coil 144. To do so, the switching circuit 142 is operable to connect the power/antenna coil 144 to a power terminal(s) or port of the transmitter circuit 140 when power is to be provided thereto and to connect the power/antenna coil 144 to an output terminal(s) or port of the transmitter circuit 140 when power is not being provided and transmission of the wireless signal is desired. For example, the switching circuit 142 may include a coil or other device responsive to the magnetic field generated by the external power source to switch the connection of the power/antenna coil 144 from the output terminal of the transmitter circuit to the power terminal. As such, when the external power source is positioned near the skin of the patient in the vicinity of the orthopaedic medical device, the power/antenna coil 144 is inductively coupled with the external power source and connected to the power terminal of the transmitter circuit 140 via the switching circuit 142. To prolong operation time without use of the external power source, the orthopaedic medical device 120 may also include an internal power source (not shown), such as a battery, that is connected to the transmitter circuit 140 to provide power thereto.

Although the embodiments of the orthopaedic medical device 120 described above with reference to FIGS. 7 and 8 each receive power via an external power source, in some embodiments, the orthopaedic medical device 120 includes an internal power source (not shown). The internal power source may be embodied as, for example, a battery or the like and electrically coupled to the transmitter circuit 130, 140 to provide power thereto. In such embodiments, a separate power coil (for example power coil 134) is not required.

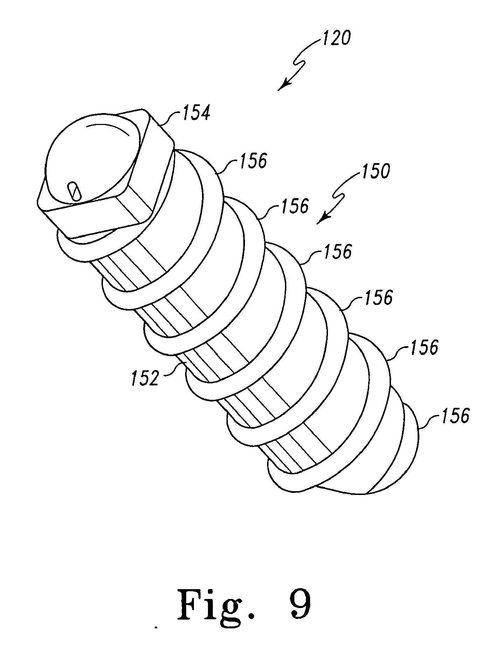

In embodiments in which the orthopaedic medical device 120 is to be coupled to a bone of the patient, the orthopaedic medical device 120 may include a housing 150 configured to be implanted into the bone as shown in FIG. 9. The circuitry associated with the medical device 120 (that is, the transmitter coils 130, 40, the antenna and/or power coils 132, 134, 144, etc.) is positioned in an inner cavity (not shown) of the housing 150. The housing 150 includes a body 152, a cap 154 configured to be coupled to the body 152, and a number of threads 156 defined about the body 152. By use of the threads 156, the housing 150 may be attached to the bone of the patient by first drilling a pilot hole into the bone using a suitable orthopaedic surgical drill or the like and subsequently screwing the housing 150 into the hole created by the surgical drill. It should be appreciated, however, that the housing 150 is only one example of a housing which is capable of being coupled to a bone of a patient and that in other embodiments other housings having various configurations may be used. For example, in some embodiments, a press-fit housing may be used. Press-fit housings are typically devoid of any threads and are configured to be pressed into a hole or cavity that has been drilled or formed into the bone. Additionally, other types of housings may be used in embodiments in which the orthopaedic medical device 120 is coupled to an orthopaedic implant or an orthopaedic surgical tool.

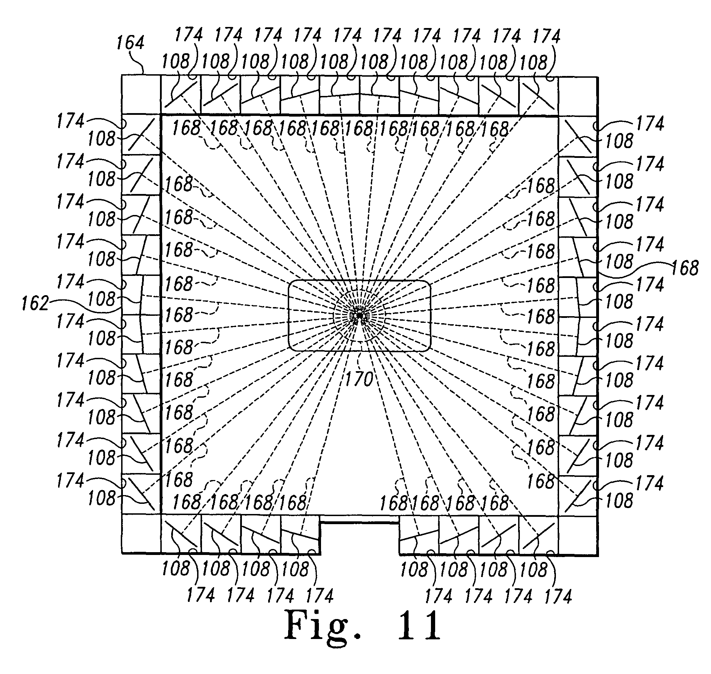

FIG. 10 shows the antenna array 104 incorporated into an orthopaedic surgery operating room 160. The antennae 108 of the antenna array 104 are coupled to one or more walls of the operating room 160 coplanar with each other so as to define a reference plane. As shown in FIG. 11, the antennae 108 are coupled to the walls 162, 164, 166 such that the boresight 168 of each antenna 108 is directed toward a common volume of space 170 of the operating room 160 in which the orthopaedic surgery procedure is to be performed. During the performance of the orthopaedic surgery procedure, the patient 56 or relevant portion of the patient 56 is positioned within the common volume 170. For example, the antennae 108 may be coupled to the walls 162, 164, 166 such that the boresight 168 of each antenna 108 is directed toward an orthopaedic operating table 172 positioned in the operating room 160.

As shown in FIG. 11, the walls 162, 164, 166 may include recesses 174 in which the antennae 108 are positioned. The antennae 108 located farther from the center area of the associated wall 162, 164, 166 are angled to a greater degree than the antennae 108 located toward the center area of the associated wall 162, 164, 166 such that the boresight 168 of each antenna 108 is directed toward the common volume 170 and/or operating table 172. In some embodiments, a radio frequency permeable window or panel 174 is coupled to the walls 162, 164, 166 in front of the antennae 108 such that the antennae 108 are hidden from view as shown in FIG. 10. In embodiments in which the antennae 108 are directional antennae, the antennae 108 are more sensitive to wireless signals transmitted from sources positioned in the common volume 170 and less sensitive to wireless signals transmitted from sources positioned outside of the common volume 170.

Referring back to FIG. 10, the antennae 110 of the antenna array 104 are coupled to a support structure 180 secured to a ceiling of the operating room 160 via a number of support arms 182. The antennae 110 are positioned such that the antennae 110 are non-coplanar with respect to the antennae 108. The antennae 110 are coupled to the support structure 180 such that each antenna 110 is directed toward the common volume of space 170. For example, the antennae 110 may be coupled to the support structure 180 or otherwise positioned such that a boresight of each antenna 110 is directed to the common volume of space 170. To do so, the antennae 110 may be coupled to an inner side 184 of the support structure 180. Because the inner side 184 of the support structure 180 is substantially inward curving, each of the antennae 110 may be positioned so as to be directed to the common volume of space 170 and/or operating table 172. In one embodiment, the support structure 180 includes a number of recesses defined in the inner side 184. In such an embodiment, the antennae 110 may be positioned therein and a radio frequency permeable window or panel 186 may be secured to the support structure 180 in front of the antennae 110.

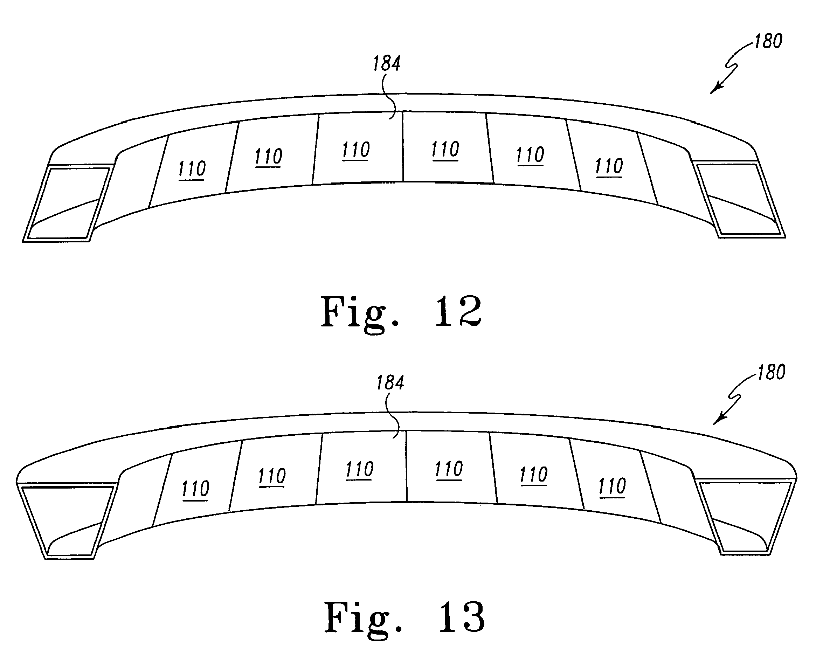

The support structure 180 may be of any configuration that facilitates the directing of the antennae 110 toward the common volume of space 170 and/or operating table 172. For example, the inner side 184 of the support structure 180 may be configured to extend outwardly in a downward direction such that each antenna 110 coupled to the inner side 184 of the support structure is directed downwardly toward the common volume of space 170 and/or operating table 172. As shown in FIG. 12, the support structure 180 has a cross-section shaped approximately so that it has the shape of a parallelogram, such that the inner side 184 extends outwardly in the downward direction. As such, the antennae 110 coupled to the inner side 184 of the support structure 180 are each angled toward the common volume of space 170. Alternatively, as shown in FIG. 13, the support structure 180 has a substantially trapezoidal cross-section such that the inner side 184 extends outwardly in the downward direction. Again, the antennae 110 coupled to the inner side 184 of the support structure 180 of FIG. 13 are each angled toward the common volume of space 170.

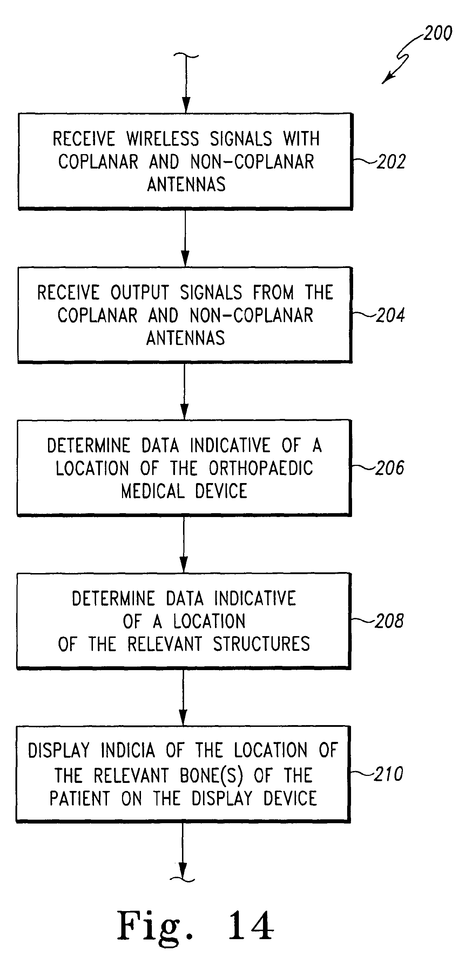

It should be appreciated that although the antenna array 104 is shown in FIGS. 10 to 13 as having many antennae 108, 110, the antenna array 104 may have more or less antennae 108, 110 in other embodiments. For example, in one embodiment, the antenna array 104 may include only three antennae 108 positioned coplanar with respect to each other so as to define a reference plane. Additionally, the antenna array 104 may include only one antenna 110 positioned non-coplanar with respect to the antennae 108. However, it should be appreciated that by including a larger number of antennae 108, 110, an amount of redundancy is provided. As such, should one or more of the antennae 108, 110 be obscured from receiving the wireless signal from the orthopaedic medical device 120 by, for example, intervening objects such as the surgeon 50 or operating room equipment, the controller 102 may still receive output signals from other non-obscured antennae 108, 110. The controller 102 can thereby still determine the location of the orthopaedic medical device 120 as discussed in more detail below with reference to FIG. 14. In addition, although the antennae 108, 110 are shown in FIGS. 10 to 13 as being coupled to the walls 162, 164, 166 and ceiling of the orthopaedic surgery operating room, in other embodiments, the antennae 108, 110 may be coupled to movable support structures similar to, for example, the stand 20 in and described above with reference to FIG. 1. In such embodiments, the antennae 108, 110 may be moved about the operating room to avoid obstruction of the wireless signal. Additionally, the antennae 108, 110 may be transported between and used in different operating rooms. However, it should be noted, that in such embodiments, the antennae 108 are positioned such that each antenna 108 is coplanar with respect to each other and that the antennae 110 are positioned non-coplanar with respect to the antennae 108.

In use, a surgeon may use the computer assisted orthopaedic surgery (CAOS) system 100 to track the location of the orthopaedic medical device(s) 120 and, thereby, the location of the patient's relevant bone(s), orthopaedic implant, and/or orthopaedic surgical tool 122 coupled thereto. To do so, the computer assisted orthopaedic surgery (CAOS) system 100 and/or the controller 102 may execute an algorithm 200 for determining the location of the orthopaedic medical device 120 and any associated structure coupled thereto. The algorithm 200, or portions thereof, may be embodied as software/firmware code stored in, for example, the memory device 114. The algorithm 200 begins with a process step 202 in which the wireless signal(s) transmitted by the orthopaedic medical device(s) 120 are received by the antennae 108, 110. As discussed above, a large number of antennae 108, 110 may be used in some embodiments to provide an amount of redundancy and improve the calculation of the location of the orthopaedic medical device(s) 120. Subsequently, in process step 204, the controller 102 receives the output signals of each of the antennae 108, 110 via the communication link 106.

Next, in process step 206, the controller 102 determines data indicative of the location of the orthopaedic medical device 120 based on the output signals received from the antennae 108, 110. Because each of the antennae 108, 110 is positioned at a different location with respect to the orthopaedic medical device 120, the output signals received from each antenna 108, 110 are different to varying amounts. As such, the location of the orthopaedic medical device 120 may be determined by comparing a portion or all of the output signals received form the antennae 108, 110. To do so, the controller 102 may execute a radio frequency direction finding algorithm. The controller 102 may use any radio frequency direction finding algorithm capable of determining data indicative of the location of the orthopaedic medical device 120 based on the output signals. For example, the controller 102 may determine the location of the orthopaedic medical device 120 by comparing or otherwise analysing the amplitudes of the various output signals, the phase of the output signals, the Doppler frequency shift of the output signals, the differential time of arrival of the output signals, and/or any other radio frequency direction finding methodology usable to determine the location of the orthopaedic medical device 120.

Once the location of the orthopaedic medical device 120 has been determined in process step 206, the location of the structure (for example patient's bone(s), orthopaedic implant, orthopaedic surgical tool, etc.) to which the orthopaedic medical device 120 is coupled is determined in process step 208. For example, in embodiments in which the orthopaedic medical device 120 is coupled to a bone of the patient, data indicative of the location of the patient's bone is determined in process step 208 based on the location of the orthopaedic medical device 120. To do so, the controller 102 may use any registration method. For example, in some embodiments, a registration tool similar to registration tool 80 is used to register the patient's bone, the orthopaedic implant, and/or the surgical tool to the controller 102. In other embodiments, pre-operative images of the patient's relevant bones, the orthopaedic implant, and/or the surgical tool having indicia of the implanted orthopaedic medical device(s) 120 are used. Based on such pre-operative images and the determined location of the orthopaedic medical device 120, the controller 102 may determine the location of the relevant bone of the patient. Subsequently in process step 210, the controller 102 displays indicia of the location of the relevant bone(s) of the patient on the display device 116. For example, the controller 102 may display a rendered image of the patient bone on the display device 116 in a location as determined in process step 208. In embodiments in which the pre-operative images are two dimensional images such as, for example, X-rays, the controller 102 may execute an appropriate two dimensional-to-three dimensional morphing algorithm to transform the two-dimensional image of the patient's bone to a three-dimensional image and display such three-dimensional image to the surgeon 50 on the display device 116 based on the determined location of the bone.

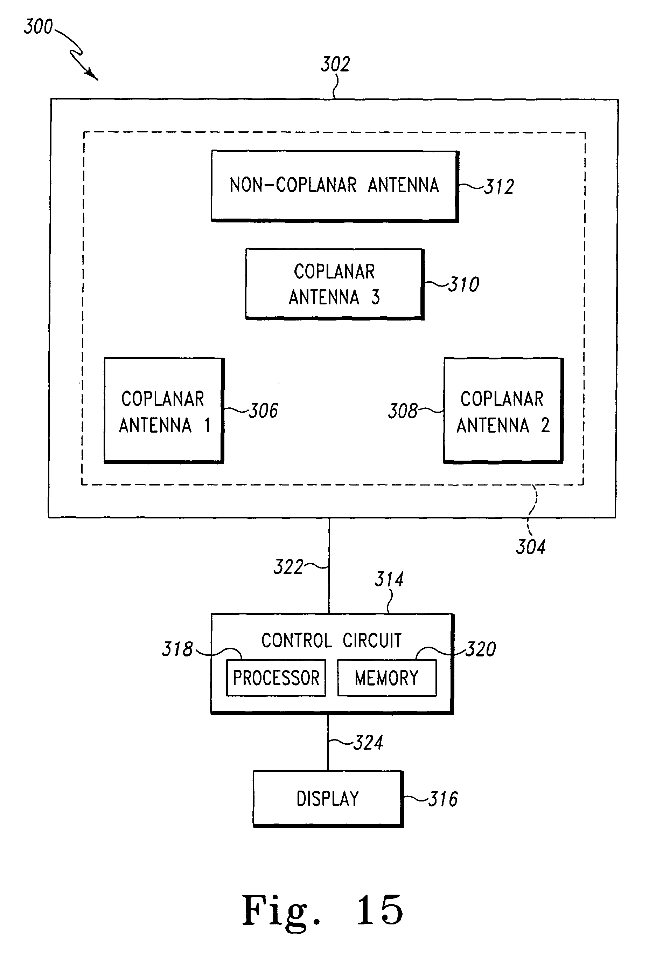

Referring now to FIG. 15, in another embodiment, a system 300 for monitoring kinematic motion of a patient includes a patient exercise machine 302, an antenna array 304 coupled to the patient exercise machine 302, a controller 314, and a display device 316. The patient exercise machine 302 may be embodied as any type of exercise machine on which the patient may exercise and via which an orthopaedic surgeon or healthcare provider may observe the kinematic motion of the patient. For example the patient exercise machine 302 may be embodied as a treadmill, a stairstepper machine, a stationary bicycle, an elliptical trainer, a rowing machine, a ski machine, or the like.

The antenna array 304 includes a first antenna 306, a second antenna 308, and a third antenna 310 coupled to the patient exercise machine 302 such that each of the antennae 306, 308, 310 is coplanar with each other. The antenna array 304 also includes a fourth antenna 312 coupled to the patient exercise machine 302 such that the fourth antenna 312 is non-coplanar with respect to the antennae 306, 308, 310. In one embodiment, the antennae 306, 308, 310, 312 are directional antennae such as, for example, spiral directional antennae. The antennae 306, 308, 310, 312 are positioned such that each of the antennae 306, 308, 310, 312 is directed toward the patient exercise machine 302 and, more specifically, toward a volume of space occupied by the relevant portion of the patient when the patient is exercising on the patient exercise machine 302. For example, the antennae 306, 308, 310 may be positioned such that the boresights of the antennae 306, 308, 310 define a reference plane. The antenna 312 may be positioned off of but directed toward the reference plane such that the boresight of the antenna 312 intersects the reference plane defined by the antennae 306, 308, 310.

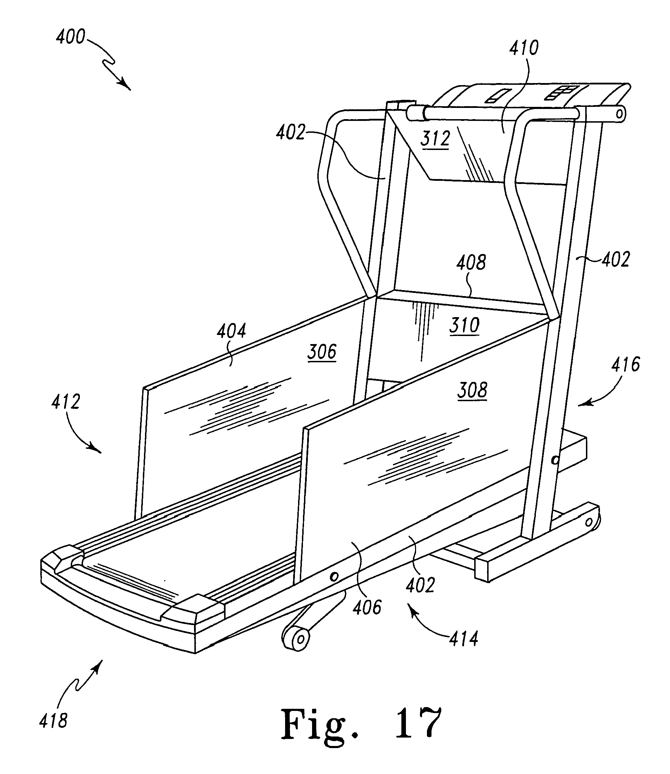

FIG. 17shows a patient exercise machine 302 in the form of a treadmill 400. As discussed above, the coplanar antennae 306, 308, 310, 312 are coupled to the treadmill 400. To do so, the antennae 306, 308, 310, 312 are positioned in housings 404, 406, 408, 410, respectively, which are coupled to a frame 402 of the treadmill 400. That is, the first housing 404, and thereby the coplanar antenna 306, is coupled to the frame 402 of the treadmill 400 on a first longitudinal side 412. The second housing 406, and thereby the coplanar antenna 308, is coupled to the frame 402 on a second longitudinal side 414 of the treadmill 400. The third housing, and thereby the coplanar antenna 308, is coupled to the frame 402 on a front side 416 of the treadmill 400. The housings 404, 406, 408 are coupled to the frame 402 such that the antennae 306, 308, 310 are positioned coplanar with respect to each other. Additionally, the antennae are positioned such that the boresight of each antenna 306, 308, 310 is directed inwardly toward the patient exercise machine 302. That is, the antenna 306 is positioned such that the boresight of the antenna 306 is directed toward the opposite longitudinal side 414 of the treadmill. Similarly, the antenna 308 is positioned such that the boresight of the antenna 308 is directed toward the opposite longitudinal side 412. The antenna 310 is positioned such that the boresight of the antenna 310 is directed toward a rear side 418 of the treadmill 400. As such, the beamwidths of the antennae 302, 308, 310 define a common volume of space in which the relevant portion(s) of the patient is positioned when the patient is exercising on the treadmill 400. For example, if the relevant portion of the patient is a knee area, the antennae 302, 308, 310 are positioned such that the relevant knee and surrounding area of the patient is positioned in the common volume of space defined by the beamwidths of the antennae 302, 308, 310. To facilitate various areas of interest of the patient, in some embodiments, the housings 404, 406, 408 are movably coupled to the frame 402 such that the housings 404, 406, 408 may be moved to different positions to thereby move the common volume of space such that the relevant portion of the patient is positioned therein. For example, the housings 404, 406, 408 may be movably coupled to the frame 402 such that the housings 404, 406 408 may be moved vertically up or down as required based on the particular orthopaedic surgical procedure being performed and the geometries of the patient.

Further, the housing 410 is coupled to the frame 402 such that the antenna 312 is positioned non-coplanar with respect to the antennae 306, 308, 310 but is directed toward the reference plane defined by the antennae 306, 308, 310. That is, the antenna 312 is coupled to the frame 402 such that the beamwidth of the antenna 312 is directed toward the common volume of space defined by the beamwidths of the antennae 306, 308, 310. Similar to the housings 404, 406, 408, the housing 410 may be movably coupled to the frame 402 such that the housing 410 may be moved to different positions to thereby move the common volume of space such that the relevant portion of the patient is positioned therein.

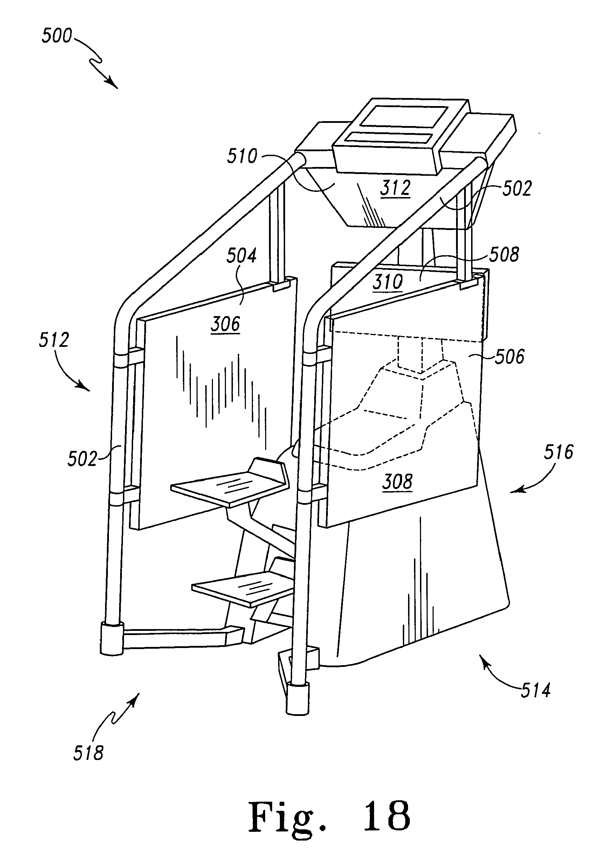

FIG. 18 shows a patient exercise machine 302 in the form of a stairstepper 500. The coplanar antennae 306, 308, 310, 312 are positioned in housings 504, 506, 508, 510, respectively, which are coupled to a frame 402 of the stairstepper 500 in a similar manner as described above with reference to the treadmill 400. That is, the first housing 504, and thereby the coplanar antenna 306, is coupled to the frame 502 of the stairstepper 500 on a first longitudinal side 512. The second housing 506, and thereby the coplanar antenna 308, is coupled to the frame 502 on a second longitudinal side 514 of the stairstepper 500. The third housing 310, and thereby the coplanar antenna 308, is coupled to the frame 502 on a front side 516 of the stairstepper 500. The housings 504, 506, 508 are coupled to the frame 502 such that the antennae 306, 308, 310 are positioned coplanar with respect to each other and the boresight of each antenna 306, 308, 310 is directed inwardly toward the patient exercise machine 302 as described above in relation to the treadmill 500. That is, the antenna 306 is positioned such that the boresight of the antenna 306 is directed toward the opposite longitudinal side 514 of the stairstepper 500. The antenna 308 is positioned such that the boresight of the antenna 308 is directed toward the opposite longitudinal side 512 and the antenna 310 is positioned such that the boresight of the antenna 310 is directed toward a rear side 518 of the stairstepper 500. As such, the beamwidths of the antennae 302, 308, 310 define a common volume of space in which the relevant portion(s) of the patient is positioned when the patient is exercising on the stairstepper 500. Similar to the treadmill 400 described above with reference to FIG. 17, the housings 504, 506, 508 may be movably coupled to the frame 502 such that the housings 404, 406, 408 may be moved to different positions to thereby move the common volume of space such that the relevant portion of the patient is positioned therein.

Additionally, the housing 510 is coupled to the frame 502 such that the antenna 312 is positioned non-coplanar with respect to the antennae 306, 308, 310 but is directed toward the reference plane defined by the antennae 306, 308, 310. That is, the antenna 312 is coupled to the frame 502 such that the beamwidth of the antenna 312 is directed toward the common volume of space defined by the beamwidths of the antennae 306, 308, 310. Similar to the housings 504, 506, 508, the housing 510 may be movably coupled to the frame 502 such that the housing 510 may be moved to different positions to thereby move the common volume of space such that the relevant portion of the patient is positioned therein.

Referring back to FIG. 15, the controller 314 includes a processor 318 and a memory device 320. The processor 314 may be embodied as any type of processor including, for example, discrete processing circuitry (for example a collection of logic devices), general purpose integrated circuit(s), and/or application specific integrated circuit(s) (ASICs). The memory device 320 may be embodied as any type of memory device and may include one or more memory types, such as, random access memory (RAM) and/or read-only memory (ROM). In addition, the controller 314 may include other devices and circuitry typically found in a computer for performing the functions described herein such as, for example, a hard drive, input/output circuitry, and the like.

The controller 314 is connected to the antenna array 304 via a number of communication links 322. The communication links 322 may be embodied as any type of communication links capable of facilitating electrical communication between the controller 314 and the antenna array 304. For example, the communication links may be embodied as any number of wires, cables, or the like. The controller 314 is also communicatively coupled with a display device 316 via a communication link 324. Although shown in FIG. 15 as separate from the controller 314, the display device 316 may form a portion of the controller 314 in some embodiments. Additionally, in some embodiments, the display device 316 or an additional display device may be positioned away from the controller 314. Additionally or alternatively, the display device 316 may be embodied as a virtual display such as a holographic display, a body mounted display such as a heads-up display, or the like. The controller 314 may also be coupled with a number of input devices such as a keyboard and/or a mouse for providing data input to the controller 314. However, in the described embodiment, the display device 316 is a touch-screen display device capable of receiving inputs from the orthopaedic surgeon 50 similar to the display device 44 described above with reference to FIG. 2. That is, the surgeon 50 can provide input data to the controller 314, such as making a selection from a number of on-screen choices, by simply touching the screen of the display device 316.

In use, a surgeon may use the system 300 to track the location of the orthopaedic medical device(s) 120 and, thereby, the location of the patient's relevant bone(s) and/or orthopaedic implant. To do so, the system 300 and/or the controller 314 may execute an algorithm 350 for monitoring the kinematic motion of a patient as defined by the motion of relevant bones of the patient. The algorithm 350, or portions thereof, may be embodied as software/firmware code stored in, for example, the memory device 320. The algorithm 350 begins with a process step 352 in which the wireless signal(s) transmitted by the orthopaedic medical device(s) 120 are received by the antennae 306, 308, 310, 312 while the patient is exercising on the patient exercise machine 302. As described above with reference to FIGS. 7 and 8, the orthopaedic medical device(s) 120 may be powered by an external transcutaneous power source such as an external primary coil or by an internal power source such as a battery or the like.

After the wireless signal(s) transmitted by the orthopaedic medical device(s) 120 are received by the antennae 306, 308, 310, 312, the controller 314 receives the output signals of each of the antennae 306, 308, 310, 312 via the communication links 322 in process step 354. Next, in process step 356, the controller 314 determines data indicative of the location of the orthopaedic medical device(s) 120 based on the output signals received from the antennae 306, 308, 310, 312. Because each of the antennae 108, 110 is positioned at a different location with respect to the orthopaedic medical device 120, the output signals received from each antenna 306, 308, 310, 312 are different to varying amounts. As such, the location of the orthopaedic medical device 120 may be determined by comparing the output signals received from the antennae 306, 308, 310, 312. To do so, similar to the controller 102 described above with reference to FIG. 6, the controller 314 may execute a radio frequency direction finding algorithm. The controller 314 may use any radio frequency direction finding algorithm capable of determining data indicative of the location of the orthopaedic medical device 120 based on the output signals. For example, the controller 314 may determine the location of the orthopaedic medical device 120 by comparing or otherwise analysing the amplitudes of the various output signals, the phase of the output signals, the Doppler frequency shift of the output signals, the differential time of arrival of the output signals, and/or any other radio frequency direction finding methodology usable to determine the location of the orthopaedic medical device 120.

Once the location of the orthopaedic medical device 120 has been determined in process step 356, the location of the patient's bone(s) to which the orthopaedic medical device(s) 120 is coupled is determined in process step 358. To do so, the controller 314 may use, for example, pre-operative images and/or post-operative images of the patient's relevant bones having indicia of the implanted orthopaedic medical device 120. Based on such pre-operative images and the determined location of the orthopaedic medical device 120, the controller 314 may determine the location of the relevant bone of the patient. Subsequently in process step 360, the controller 314 displays indicia of the location of the relevant bone(s) of the patient on the display device 316. For example, the controller 314 may display a rendered image of the patient bone on the display device 316 in a location as determined in process step 358. Alternatively, in embodiments in which basic kinematic motion is desired, the controller 314 may be configured to display line segments indicative of the relative positions of the patient's bone(s). Additionally, in some embodiments, the controller 314 may be configured to store the location data in a storage device (not shown) or the memory device 320 such that the data may be retrieved at a later time and view in sequential animation such that the range of kinematics motion of the patient may be viewed via the display device 316.

It should be appreciated that the system 300 may be used to monitor the pre-operative and/or post-operative kinematic motion of the patient. For example, prior to the orthopaedic surgical procedure, one or more orthopaedic medical devices 120 may be implanted into the relevant bones of the patient. The pre-operative kinematic motion the patient may then be determined using the system 300 and algorithm 350. The sequential location data of the patient's bones may then be stored. The orthopaedic surgical procedure may subsequently be performed and the post-operative kinematic motion of the patient may be determined using the system 300. Because the pre-operative kinematic data may be stored, the surgeon or other healthcare provided may comparatively view the kinematic motion of the patient and, thereby, determine the success or quality of the orthopaedic surgical procedure, as well as, identify any possible orthopaedic problems which the patient may encounter. In a similar manner, the kinematic motion of the patient may be post-operatively determined over a period of time such that the "wear" of an orthopaedic implant may be determined and possibly corrected for in further orthopaedic surgical procedures.

It should be appreciated that although the systems 100, 300 and algorithms 200, 350 have been described above as generally using a single orthopaedic medical device 120, any number of orthopaedic medical devices 120 may be used. For example, two or more orthopaedic medical devices 120 may be coupled to the relevant patient's bone, orthopaedic implant, or surgical tool. The location and orientation of the patient's bone, orthopaedic implant, and/or surgical tool may be determined based on the wireless signals transmitted from the plurality of orthopaedic medical devices 120. Moreover, three orthopaedic medical devices 120 may be coupled to the relevant patient's bone, orthopaedic implant, or surgical tool. If so, the six degrees of freedom of the patient's bone, orthopaedic implant, or surgical tool may be determined based on the wireless signals transmitted from the three orthopaedic medical devices 120. However, it should be appreciated that the six degrees of freedom of the relevant patient's bone, orthopaedic implant, and/or surgical tool may be determined using more or less orthopaedic medical devices 120.

As discussed above with reference to FIGS. 6 and 15, the computer assisted orthopaedic surgery (CAOS) system 100 and/or the system 300 may include any number of orthopaedic medical devices 120 in some embodiments. In embodiments in which the orthopaedic medical devices 120 are embodied as the devices 120 described above with reference to FIGS. 7 and 8, the orthopaedic medical devices 120 are configured to transmit non-modulated wireless signals using different frequencies. However, in other embodiments, each of the orthopaedic medical devices 120 may be configured to transmit a modulated signal using the same carrier frequency. In such embodiments, the orthopaedic medical devices 120 transmit a serial number associated with each respective device 120.

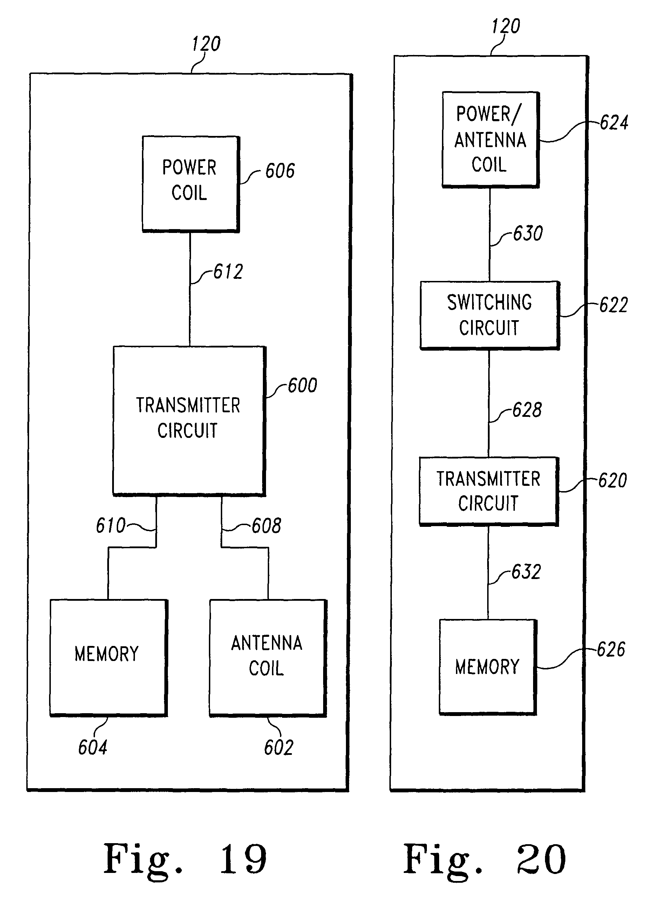

For example, as shown in FIG. 19, the orthopaedic medical device(s) 120 can include a transmitter circuit 600, an antenna coil 602, a memory device 604, and a power coil 606. The transmitter circuit 600 is communicatively coupled to the antenna coil 602 via a number of communication links 608, to the memory device 604 via a number of communication links 610, and to the power coil 606 via a number of communication links 612. The communication links 608, 610, 612 may be embodied as any type of communication links capable of facilitating communication between the transmitter circuit 600 and the antenna coil 602, the memory device 604, and the power coil 606, respectively. For example, the communication links 608, 610, 612 may be embodied as wires, cables, printed circuit board (PCB) traces, fiber optic cables, or the like.

The transmitter circuit 600 may be embodied as or include any type of transmitter circuitry capable of generating a modulated wireless signal using a predetermined carrier frequency. For example, the transmitter circuit 600 may include a simple inductor-capacitor (LC) circuit or a crystal oscillator circuit and associated circuitry. In addition, the transmitter circuit 600 includes circuitry for accessing data stored in the memory device 604. The memory device 604 may be embodied as any type of memory device and may include one or more memory types, such as, random access memory (RAM) and/or read-only memory (ROM). In one particular embodiment, the memory device 604 has stored therein and/or is capable of storing a serial number associated with the respective orthopaedic medical device 120. The serial number may be embodied as any type of data that uniquely identifies the respective orthopaedic medical device 120 from other orthopaedic medical devices 120 being used in the relevant orthopaedic medical procedure. In addition, in some embodiments, an error correction code (ECC) associated with the serial number may also be stored in the memory device 120 such that the respective serial number may be validated by the controller 102/control circuit 314. Alternatively, the transmitter circuit 600 may include additional processing circuitry capable of determining the error correction code from the serial number such that it is not required to store the error correction code in the memory device 120 prior to transmission of the serial number. The error correction code may be embodied as any type of data capable of providing validation of the accuracy of the serial number once received by the controller 102/control circuit 314. In some embodiments, the error correction code may be embodied as, for example, a checksum or a cyclic redundancy check value or data.

In operation, the transmitter circuit 600 is configured to retrieve the serial number from the memory device 604 via the communication link 610. In addition, the transmitter circuit 600 retrieves the error correction code from the memory device 604 or, in some embodiments, computes the error correction code based on the retrieved serial number. Regardless, the transmitter circuit 130 transmits the serial number and error correction code via the communication link 608 and the antenna coil 602.

The transmitter circuit 600 transmits the serial number and error correction code via use of a predetermined carrier frequency. That is, the wireless signal generated by the transmitter circuit 130 and antenna coil 602 is a modulated wireless signal. As discussed above, in embodiments in which multiple orthopaedic medical devices 120 are used, each orthopaedic medic device 120 is configured to transmit the modulated wireless signal using a single predetermined carrier frequency. Such a predetermined frequency may be embodied as any frequency receivable by the relevant antenna array 104, 304. In one embodiment, the transmitter circuit 130 is configured to transmit the wireless signals in the very-high frequency (VHF) band or ultra-high frequency (UHF) band. Because a single predetermined carrier frequency is used by each of the orthopaedic medical devices 120, each of the antennae of the antenna array 104, 304 may be tuned or otherwise optimized to receive the predetermined frequency.

In some embodiments, each orthopaedic medical device 120 is configured to transmit the respective serial number and error correction code at a different pulse repetition frequency (PRF) to ensure that the controller 102/control circuit 314 is capable of distinguishing between the multiple signals. That is, the orthopaedic medical devices 120 are configured to transmit at different periods of time such that no two or more devices 120 are transmitting at the same time.

The transmitter circuit 600 receives power via the power coil 606. The power coil 606 is configured to be inductively coupled to a power source (not shown) external to the patient. The power coil 606 may include any number of individual coils. For example, the power coil 606 may include a single coil that is inductively coupled to the external power source by positioning the external power source near the skin of the patient such that the power coil 606 lies within an alternating current (AC) magnetic field generated by the external power source. In other embodiments, the power coil 606 includes more than a single coil to thereby improve the inductive coupling of the power coil 606 and the external power source. That is, because the amount of inductive coupling of the power coil 606 and the external power source is dependent upon the alignment of the power coil 606 and the magnetic field generated by the external power source, a power coil having multiple coils at different orientations decreases the likelihood of poor inductive coupling with the external power source. For example, in one embodiment, the power coil 606 is embodied as three separate coils positioned orthogonally with respect to each other. The external power source may be embodied as any type of power source capable of inductively coupling with the power coil 606 and generating a current therein. In one embodiment, the external power source includes two patches couplable to the skin of the patient in the vicinity of the orthopaedic medical device 120. The patches each include a Helmholtz-like coil and are powered such that the Helmholtz coils produce an isotropic magnetic field, which is received by the power coil 134.

FIG. 20 shows an orthopaedic medical device 120 which includes a transmitter circuit 620, a switching circuit 622, a power/antenna coil 624, and a memory device 626. The transmitter circuit 620 is communicatively coupled to the switching circuit 622 via a number of communication links 628 and to the memory device 626 via a number of communication links 632. The switching circuit 622 is coupled to the power/antenna coil 624 via a number of communication links 630. Similar to the communication links 608, 610, 612 described above with reference to FIG. 19, the communication links 628, 630, 632 may be embodied as any type of communication links capable of facilitating communication between the transmitter circuit 620, the switching circuit 622, the power/antenna coil 624, and the memory device 626. For example, the communication links 628, 630, 632 may be embodied as wires, cables, printed circuit board (PCB) traces, fiber optic cables, or the like.

The transmitter circuit 620 is generally similar to the transmitter 600 described above with reference to FIG. 19 and, as such, may be embodied as or include any type of transmitter circuit capable of generating a modulated wireless signal using a predetermined carrier frequency. For example, the transmitter circuit 620 may include a simple inductor-capacitor (LC) circuit or a crystal oscillator circuit and associated circuitry. In addition, similar to transmitter 600, the transmitter circuit 620 includes circuitry for accessing data stored in the memory device 626. The memory device 626 may be embodied as any type of memory device and may include one or more memory types, such as, random access memory (RAM) and/or read-only memory (ROM). In one particular embodiment, the memory device 626 has stored therein and/or is capable of storing a serial number associated with the particular orthopaedic medical device 120. As discussed above, the serial number may be embodied as any type of data capable of uniquely identifying the particular orthopaedic medical device 120 from other orthopaedic medical devices 120 being used in the relevant orthopaedic surgical procedure. Additionally, an error correction code associated with the serial number may also be stored in the memory device 626. Alternatively, the transmitter circuit 620 may include additional processing circuitry capable of determining the error correction code from the serial number. As discussed above with reference to FIG. 19, the error correction code may be a embodied as any type of data capable of providing validation of the accuracy of the serial number once received by the controller 102/control circuit 314.

Similar to the transmitter circuit 600, the transmitter circuit 620 is configured to retrieve the serial number and, in some embodiments, the error correction code from the memory device 626 via the communication link 632 and transmit the serial number and error correction code using the switching circuit 622 and the power/antenna coil 624 as discussed below. The transmitter circuit 620 transmits the serial number and error correction code via use of a predetermined carrier frequency. Similar to transmitter circuit 600, when multiple devices 120 are used during an orthopaedic medical procedure, each orthopaedic medic device 120 is configured to transmit the modulated wireless signal using a single predetermined carrier frequency. Again, the predetermined frequency may be embodied as any frequency receivable by the relevant antenna array 104, 304. For example, in one embodiment, the transmitter circuit 620 is configured to transmit the wireless signals in the very-high frequency (VHF) band or ultra-high frequency (UHF) band. Additionally, in some embodiments, each orthopaedic medical device 120 is configured to transmit the respective modulated wireless signal at a different pulse repetition frequency (PRF) to ensure that the controller 102 or control circuit 314 is capable of distinguishing between the multiple signals.

In the embodiment shown in FIG. 20, the transmitter circuit 620 receives power and transmits a modulated wireless signal using the same coil, that is, the power/antenna coil 624. To do so, the switching circuit 622 is operable to connect the power/antenna coil 624 to a power terminal(s) or port of the transmitter circuit 620 when power is to be provided thereto and to connect the power/antenna coil 624 to an output terminal(s) or port of the transmitter circuit 620 when power is not being provided and transmission of the modulated wireless signal is desired. For example, the switching circuit 622 may include a coil or other device responsive to the magnetic field generated by the external power source to switch the connection of the power/antenna coil 624 from the output terminal of the transmitter circuit to the power terminal. As such, when the external power source is positioned near the skin of the patient in the vicinity of the orthopaedic medical device, the power/antenna coil 624 is inductively coupled with the external power source and connected to the power terminal of the transmitter circuit 620 via the switching circuit 622.

Although the embodiments of the orthopaedic medical device 120 described above with reference to FIGS. 19 and 20 each receive power via an external power source, in some embodiments, the orthopaedic medical device 120 includes an internal power source (not shown). The internal power source may be embodied as, for example, a battery or the like and electrically coupled to the transmitter circuit 130, 140 to provide power thereto. In such embodiments, a separate power coil (for example power coil 606) is not required.

As discussed above with reference to FIGS. 7 and 8, the circuitry associated with the medical device 120 (that is, the transmitter circuit 600, 620, memory device 604, 626, antenna and/or power coils 602, 606, 624, etc.) may be positioned in a housing, such as housing 150 shown in FIG. 9, in embodiments in which the device 120 is to be coupled to a bone of a patient. Again, it should be appreciated, that the housing 150 is but only one example of housings which are capable of being coupled to a bone of a patient and that in other embodiments other housings having various configurations may be used. For example, in some embodiments, a press-fit housing may be used. Press-fit housings are typically devoid of any threads and are configured to be pressed into a hole or cavity that has been drilled or formed into the bone. Additionally, other types of housings may be used in embodiments in which the orthopaedic medical devices 120 shown in FIGS. 19 and 20 are coupled to an orthopaedic implant or an orthopaedic surgical tool.

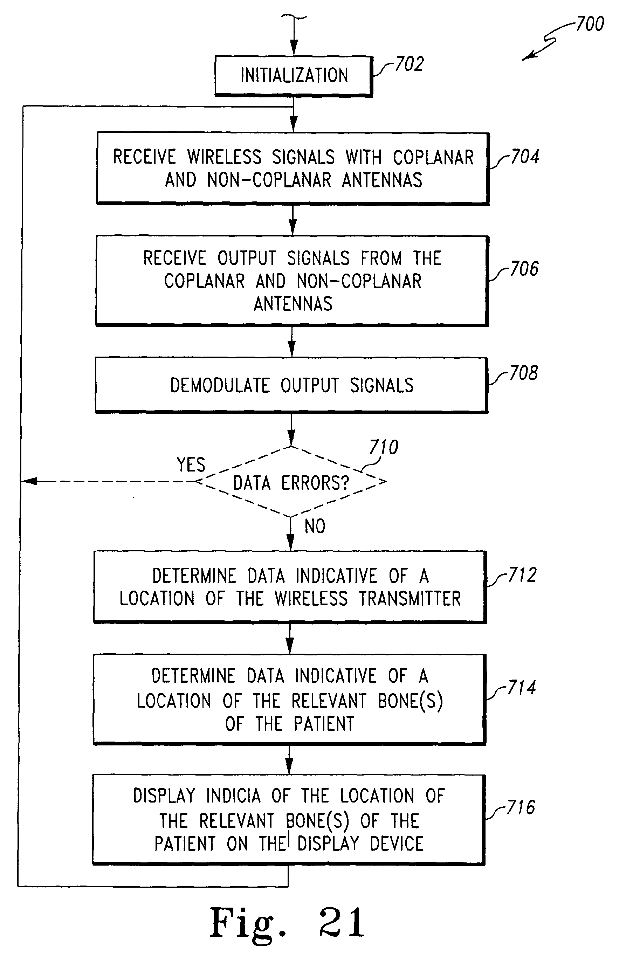

Referring now to FIG. 21, in embodiments in which the orthopaedic medical device(s) 120 are embodied as the devices 120 described above with reference to FIGS. 19 and 20, the computer assisted orthopaedic surgery (CAOS) system 100 (for example the controller 102) and/or the system 300 (for example the control circuit 314) may execute an algorithm 700 for determining the location of the orthopaedic medical device(s) 120 and any associated structure coupled thereto. The algorithm 700, or portions thereof, may be embodied as software/firmware code stored in, for example, the memory device 114, 320. The algorithm 700 will be described below in reference to the system 100 shown in FIG. 6 with the understanding that the algorithm 700 may be used by other systems such as the system 300 described above with reference to FIG. 15.

The algorithm 700 begins with a process step 702 in which the system 100 is initialized. The initialization process may include, for example, selecting user preferences on the controller 102, assigning base values to variables, verification of operation of the orthopaedic medical devices 120, antenna array 104, and/or controller 102, and/or the like. In addition, the serial number of each orthopaedic medical device 120 used in the orthopaedic medical procedure is matched to respective images or other displayable indicia of the orthopaedic medical device 120. For example, in some embodiments, a "look-up" data table or other type of data set is used to cross-reference the serial number of each orthopaedic medical device 120 to the respective image that is displayed or will be displayed on the display device 116. In this way, the controller 102 may update the correct orthopaedic medical device image or indicia on the display device 116 as discussed in more detail below with relation to the process step 716. The "look-up" data table may be stored in, for example, the memory device 114.

Once the system 100 has been initialized in process step 702, the modulated wireless signal transmitted by one of the orthopaedic medical device 120 is received by the antennae 108, 110 in process step 704. As discussed above with reference to FIGS. 19 and 20, because each orthopaedic medical device 120 transmits a respective serial number on a predetermined carrier frequency at different pulse repetition frequency, only one orthopaedic medical device 120 will be transmitting during a given period of time. As such, the modulated wireless signal from one orthopaedic medical device 120 will be received by the antennae 108, 110 in process step 704. As discussed above with reference to FIGS. 10 to 13, a large number of antennae 108, 110 may be used in some embodiments to provide an amount of redundancy and improve the calculation of the location of the orthopaedic medical device(s) 120.

In process step 706, the controller 102 receives the output signals of each of the antennae 108, 110 via the communication link 106. Because each of the antennae 108, 110 may be located at a different distance or angle with respect to the orthopaedic medical device 120, each output signal received from the antennae 108, 110 may be different by various amounts. For example, the amplitude of the signal, the phase shift of the signal, and/or the like may be different. Once the output signals are received from the antennae 108, 110, the output signals are demodulated in process step 708. By demodulating the output signals, the controller 102 determines the serial number transmitted by the orthopaedic medical device 120. In addition, in some embodiments, the controller 102 determines the error correction code associated with the serial number and transmitted by the orthopaedic medical device 120.

In such embodiments, the controller 102 is configured to verify the validity of the determined serial number in process step 710. To do so, the controller 120 may perform calculations on the serial number and compare the result of such calculations to the serial number. The specific verification procedure used by the controller 102 in process step 710 may depend upon the type of error correction code used. For example, in more complex validation schemes such as a cyclic redundancy check scheme, a more complicated verification procedure may be used. Regardless, the controller 102 is configured to verify that the serial number determined in process step 708 is a valid serial number.

If the controller 102 determines that the serial number determined in process step 708 is not a valid serial number or otherwise contains data errors, the algorithm 700 loops back to process step 704 in which a new modulated wireless signal is received by the antennae 108, 110. In embodiments in which a single orthopaedic medical device 120 is used, the new modulated wireless signal is transmitted by the same orthopaedic medical device 120. However, in embodiments in which multiple orthopaedic medical devices 120 are used, the new modulated wireless signal may be transmitted by a different orthopaedic medical device 120. In such embodiments, the modulated wireless signal from the previous orthopaedic medical device 120 will be received at a later iteration of the algorithm 700 (that is, during the period in which the previous device 120 is configured to transmit again) as discussed below in relation to process step 716. In this way, even when a data error is encountered, the location of the orthopaedic medical device 120 may still be determined based on subsequent transmissions of the modulated wireless signal.

If, however, the controller 102 determines that the serial number is valid, the algorithm 700 advances to process step 712. In process step 712, the controller 102 determines data indicative of the location of the orthopaedic medical device 120 based on the demodulated output signals (for example the serial number or data signal indicative of the serial number) determined in process step 708. Because each of the antennae 108, 110 is positioned at a different location with respect to the orthopaedic medical device 120, the output signal received from each antenna 108, 110 is different to varying amounts. As such, the location of the orthopaedic medical device 120 may be determined by comparing a portion or all of the demodulated output signals (that is, the serial number or data signal indicative of the serial number) received from the antennae 108, 110. To do so, the controller 102 may execute a radio frequency direction finding algorithm. The controller 102 may use any radio frequency direction finding algorithm capable of determining data indicative of the location of the orthopaedic medical device 120 based on the demodulated output signals (for example the serial number). For example, the controller 102 may determine the location of the orthopaedic medical device 120 by comparing or otherwise analysing the amplitudes of the various demodulated output signals, the phase of the demodulated output signals, the Doppler frequency shift of the demodulated output signals, the differential time of arrival of the demodulated output signals, and/or any other radio frequency direction finding methodology usable to determine the location of the orthopaedic medical device 120.

Once the location of the orthopaedic medical device 120 has been determined in process step 712, the location of the structure (for example patient's bone(s), orthopaedic implant, orthopaedic surgical tool, etc.) to which the orthopaedic medical device 120 is coupled is determined in process step 714. To do so, the controller 102 first compares the serial number, as determined in process step 708, to the "look-up" table generated in process step 702 such that the correct image or other indicia of the orthopaedic medical device 102 may be updated and displayed. In embodiments in which the orthopaedic medical device 120 is coupled to a bone of the patient, data indicative of the location of the patient's bone is determined in process step 714 based on the location of the orthopaedic medical device 120. To do so, the controller 102 may use any registration method. For example, in some embodiments, a registration tool similar to registration tool 80 is used to register the patient's bone, the orthopaedic implant, and/or the surgical tool to the controller 102. In other embodiments, pre-operative images of the patient's relevant bones, the orthopaedic implant, and/or the surgical tool having indicia of the implanted orthopaedic medical device(s) 120 are used. Based on such pre-operative images and the determined location of the orthopaedic medical device 120, the controller 102 may determine the location of the relevant bone of the patient.

Subsequently in process step 716, the controller 102 displays or updates images or indicia of the location of the relevant bone(s) of the patient on the display device 116. For example, the controller 102 may display a rendered image of the patient bone on the display device 116 in a location as determined in process step 714. In embodiments in which the pre-operative images are two dimensional images such as, for example, X-rays, the controller 102 may execute an appropriate two dimensional-to-three dimensional morphing algorithm to transform the two-dimensional image of the patient's bone to a three-dimensional image and display such three-dimensional image to the surgeon 50 on the display device 116 based on the determined location of the bone.

Once the correct image or indicia of the structure to which the orthopaedic medical device 120 is coupled has been displayed or updated in process step 716, the algorithm 700 loops back to process step 704. In process step 704, a new modulated wireless signal is received by the antennae 108, 110. In embodiments in which a single orthopaedic medical device 120 is used, the new modulated wireless signal is transmitted by the same orthopaedic medical device 120. However, in embodiments in which multiple orthopaedic medical devices 120 are used, the new modulated wireless signal may be transmitted by a different orthopaedic medical device 120 due to the different pulse repetition frequency used by each orthopaedic medical device 120. As discussed above, in such embodiments, the location of the pervious orthopaedic medical device 120 is determined during a subsequent iteration of the algorithm 700 (that is, during a subsequent period in which the previous device 120 is configured to transmit again).

## Claims

1. A computer assisted orthopaedic surgery system comprising:
a first orthopaedic medical device having a first wireless transmitter that is configured to transmit a first wireless signal using a predetermined carrier frequency;
a second orthopaedic medical device having a second wireless transmitter that is configured to transmit a second wireless signal using the predetermined carrier frequency;
a plurality of antennae; and
a controller electrically coupled to the plurality of antennae and configured (i) to receive first output signals from the plurality of antennae in response to the first wireless signal, (ii) to receive second output signals from the plurality of antennae in response to the second wireless signal, (iii) to demodulate the first and second output signals, (iv) to determine a location of the first orthopaedic medical device based on the demodulated first output signals, and (v) to determine a location of the second orthopaedic medical device based on the demodulated second output signals.

2. The computer assisted orthopaedic surgery system of claim 1, in which at least one of the first orthopaedic medical device and the second orthopaedic medical device is coupled to an orthopaedic implant.

3. The computer assisted orthopaedic surgery system of claim 1, in which at least one of the first orthopaedic medical device and the second orthopaedic medical device is coupled to an orthopaedic surgical tool.

4. The computer assisted orthopaedic surgery system of claim 1, in which at least one of the first orthopaedic medical device and the second orthopaedic medical device is configured to be implanted into a bone of a patient.

5. The computer assisted orthopaedic surgery system of claim 1, in which the first wireless signal comprises a serial number of the first orthopaedic medical device and the second wireless signal comprises a serial number of the second orthopaedic medical device.

6. The computer assisted orthopaedic surgery system of claim 1, in which:
(i) the first orthopaedic medical device is configured to transmit the first wireless signal at a first pulse repetition frequency, and
(ii) the second orthopaedic medical device is configured to transmit the second wireless signal at a second pulse repetition frequency,
in which the first pulse repetition frequency is different from the second pulse repetition frequency.

7. The computer assisted orthopaedic surgery system of claim 1, in which the plurality of antennae comprises:
(i) a plurality of first antennae each of which is positioned substantially coplanar with each other, and
(ii) a second antenna positioned non-coplanar with respect to the plurality of first antennae.

8. The computer assisted orthopaedic surgery system of claim 7, in which the plurality of first antennae and the second antenna are spiral directional antennae.

9. The computer assisted orthopaedic surgery system of claim 7, in which the plurality of first antennae are positioned such that a boresight of each first antenna is directed toward a common volume of space and the second antenna is positioned such that a boresight of the second antenna is directed toward the common volume of space.

10. The computer assisted orthopaedic surgery system of claim 1, in which the controller is configured to:
(i) determine the location of the first orthopaedic medical device by comparing the demodulated first output signals; and
(ii) determine the location of the second orthopaedic medical device by comparing the demodulated second output signals.

11. The computer assisted orthopaedic surgery system of claim 10, in which the controller is configured to determine the location of the first and second orthopaedic medical devices using a radio frequency direction finding algorithm.

12. The computer assisted orthopaedic surgery system of claim 1, further comprising a display device electrically coupled to the controller, in which the controller is configured to display indicia of the location of the first and second orthopaedic medical devices on the display screen.

13. A computer assisted orthopaedic surgery system comprising:
an orthopaedic medical device having a wireless transmitter that is configured to transmit a modulated wireless signal;
a plurality of first antennae each of which is positioned substantially coplanar with each other;
a second antenna positioned non-coplanar with respect to the plurality of first antennae; and
a controller electrically coupled to the plurality of first antennae and the second antenna and configured to (i) receive output signals from the plurality of first antennae and the second antenna in response to the modulated wireless signal, (ii) demodulate the output signals, and (ii) determine the location of the orthopaedic medical device based on the demodulated output signals.

14. The computer assisted orthopaedic surgery system of claim 13, in which the modulated wireless signal comprises a serial number of the orthopaedic medical device.

15. The computer assisted orthopaedic surgery system of claim 13, in which the controller is configured to determine the location of the orthopaedic medical device using a radio frequency direction finding algorithm.

16. An implantable orthopaedic medical device for use in determining a location of a bone of a patient, the implantable orthopaedic medical device comprising:
a housing;
an antenna coil positioned in the housing;
a memory device positioned in the housing and having stored therein a serial number associated with the implantable orthopaedic medical device; and
a transmitter circuit positioned in the housing and electrically coupled to the antenna coil and the memory device, in which the transmitter is configured to transmit the serial number on a predetermined carrier frequency at a predetermined pulse repetition frequency using the antenna coil.

17. The implantable orthopaedic medical device of claim 16, further comprising a switching circuit coupled to the antenna coil and the transmitter circuit, the switching circuit operable to selectively electrically connect the antenna coil to a power terminal of the transmitter circuit or an output terminal of the transmitter circuit.

18. The implantable orthopaedic medical device of claim 16, further comprising a power coil electrically coupled to the transmitter circuit, in which the power coil is configured to be inductively coupled to a power source external to the patient to supply power to the transmitter circuit.
